# EUROPÄISCHE PATENTSCHRIFT

(11) **EP 1 336 384 B1**
(45) Veröffentlichungstag und Bekanntmachung des Hinweises auf die Patenterteilung: **23.07.2008**
(21) Anmeldenummer: 03009909.7
(22) Anmeldetag: 18.08.2000
(51) Int. Cl.: A61B 18/14, A61B 17/28

(54) **Medizinisches bipolares Instrument zum Schneiden von Gewebe**
Bipolar medical device for cutting tissue
Instrument medical bipolaire pour couper des tissues

(30) Priorität: 28.09.1999 DE 19946527; 14.02.2000 DE 20002645 U
(43) Veröffentlichungstag der Anmeldung: 20.08.2003
(62) Teilanmeldung aus: 00964023.6
(73) Patentinhaber: Karl Storz GmbH & Co. KG, 78532 Tuttlingen (DE)
(72) Erfinder: Blocher, Martin, 78532 Tuttlingen (DE); Remorgida, Valentino, 16145 Genua (IT)
(74) Vertreter: Heuckeroth, Volker

(56) Entgegenhaltungen:
- WO-A-99/05976
- WO-A-99/23960
- US-A- 5 637 111

## Beschreibung

Die Erfindung betrifft ein medizinisches bipolares Instrument zum Schneiden von Gewebe.

Ein medizinisches bipolares Instrument zum Schneiden von Gewebe ist aus dem DE-Firmenkatalog der Firma Karl Storz GmbH & Co. KG, Tuttlingen, "STORZ Karl Storz-Endoskope", Band "Laparoskopie", 3. Ausgabe 1/1999, Seite BI-COA 10/2, bekannt.

Ein solches medizinisches bipolares Instrument dient bspw. in der laparoskopischen Chirurgie zum Präparieren von Gewebe im menschlichen oder tierischen Körper.

Beim Präparieren von Gewebe werden in das Gewebe Schnitte eingebracht. Da beim Einbringen von Schnitten Blutungen auftreten können, müssen diese gestillt werden.

Bei dem bekannten Instrument ist die Anwendung von bipolarem Hochfrequenzstrom auf die Blutstillung, d.h. Koagulation beschränkt. Das Einbringen von Schnitten in das Gewebe erfolgt dagegen auf mechanischem Wege, und zwar mittels einer skalpellartigen Klinge.

Das zuvor genannte bekannte Instrument weist ein erstes Arbeitselement in Form eines Mauls und ein zweites Arbeitselement ebenfalls in Form eines Mauls auf, die Seite an Seite voneinander beabstandet angeordnet sind, mit anderen Worten in der Art einer Zwillingszange. Das das erste Arbeitselement bildende Maul ist als mit Hochfrequenzstrom beaufschlagbare Elektrode und das das zweite Arbeitselement bildende zweite Maul als Gegenelektrode ausgebildet, wobei das erste Maul über eine entsprechende Stromzuführung mit dem einen Pol einer Hochfrequenzspannungsquelle und das zweite Maul über eine entsprechende Stormzuführung mit dem anderen Pol der Hochfrequenzspannungsquelle verbindbar ist. Das erste Maul und das zweite Maul sind durch ihre Beabstandung voneinander gegeneinander isoliert.

Ein damit vergleichbares Instrument ist aus der WO 97/17033 bekannt.

Beim Einschalten der Hochfrequenzspannungsquelle bildet sich ein Stromfluß durch das zwischen dem ersten Maul und dem zweiten Maul befindliche Gewebe aus, so daß der Koagulationseffekt auf den Bereich zwischen den beiden Mäulern beschränkt ist. Zum Koagulieren eines Gefäßes wird dieses sich quer zu den Mäulern erstreckend gefaßt.

Die Schneidfunktion dieses bekannten Instruments zum Schneiden von Gewebe, insbesondere zum Durchtrennen von Gefäßen, die in dem Gewebe vorhanden sein können, ist bei dem bekannten Instrument durch eine skalpellartige Klinge realisiert, die zwischen dem ersten Maul und dem zweiten Maul angeordnet ist, und die über eine separate Handhabe am proximalen Ende des Instruments in Längsrichtung nach vorn geschoben werden kann, um bspw. ein Gefäß, das sich quer zu den Mäulern erstreckend gefaßt wurde, zu durchtrennen. Die Schneidwirkung der Klinge ist rein mechanisch. Durch das Vorsehen der Klinge zwischen dem ersten Maul und dem zweiten Maul wird gegenüber anderen herkömmlichen bipolaren Instrumenten, mit denen ausschließlich koaguliert werden kann, das Einführen eines zusätzlichen Schneidelements, wie eine Schere oder ein Skalpell, in das Operationsgebiet und damit ein Instrumentenwechsel vermieden.

Dennoch erweist sich die Ausgestaltung dieses bekannten Instruments mit einer skalpellartigen Klinge zum Schneiden von Gewebe als nachteilig, wenn die Abmessungen des Instruments verkleinert werden sollen, d.h. dieses möglichst schmalbauend ausgebildet werden soll. Während das bekannte Instrument einen Instrumentendurchmesser von etwa 10 mm aufweist, sind bei bestimmten Anwendungen derartiger Instrumente Durchmesser von etwa 5 mm erstrebenswert.

Eine Reduzierung des Durchmessers des bekannten Instruments von 10 mm auf 5 mm ist jedoch aus folgenden Gründen nicht möglich. Da zwischen dem ersten Maul und dem zweiten Maul die skalpellartige Klinge Platz finden muß, weisen das erste Maul und das zweite Maul des bekannten Instruments bereits einen geringen Durchmesser von nur 3 mm auf und sind etwa 3 mm voneinander beabstandet. Eine Reduzierung des Gesamtdurchmessers der Anordnung aus erstem Maul, zweitem Maul und skalpellartiger Klinge auf 5 mm würde es erfordern, das erste Maul und das zweite Maul im Durchmesser auf weniger als 1 mm zu reduzieren. Mit derartigen Abmessungen ist jedoch die Stabilität der die Arbeitselemente bildenden Mäuler nicht mehr gewährleistet. Um mit dem Instrument Gewebe sicher präparieren zu können, ist jedoch eine ausreichende Stabilität des ersten und zweiten Arbeitselements erforderlich.

Aus dem eingangs genannten DE-Firmenkatalog ist auf Seite BI-COA 5/12 ein weiteres Instrument bekannt, das ein Faß- und Koagulationsinstrument, jedoch ohne Schneidfunktion, ist, dessen Arbeitselement ein oberes und ein unteres Maulteil aufweist. Die beiden Maulteile sind als Faßwerkzeuge ausgebildet und außerdem als Elektrode und Gegenelektrode, so daß sich zwischen den Maulteilen durch zwischen diesen gefaßtes Gewebe ein Stromfluß zum Koagulieren des Gewebes ausbilden kann.

Aus der WO-A-99/23960, die den nächstliegenden Stand der Technik offenbart, ist ferner ein Instrument zum Verschweißen von Blutgefäßen bekannt. In diesem Dokument wird zum Stand der Technik eine Koagulationszange beschrieben, die ein Maul mit zwei beweglichen Maulteilen aufweist, die auf ihrer einander zugewandten Seite jeweils eine Zahnung aufweisen, um ein zu schweißendes Blutgefäß sicher fassen zu können. Das Instrument kann auch in bipolarem Modus betrieben werden, wobei dann das eine Maulteil und das andere Maulteil als Gegenelektrode wirken. In demselben Dokument ist ein weiteres Instrument zum Verschweißen von Blutgefäßen offenbart, bei dem zur Erzielung einer Schneidfunktion eine hin und her bewegbare Klinge zum Durchschneiden des verschweißten Blutgefäßes vorgesehen ist, mit der demnach mechanisch geschnitten wird. Alternativ dazu kann auch eine separate scherenartige Klinge vorgesehen sein, die in einem Schlitz in einem Maulteilarm drehbar ist. Auch bei diesem bekannten Instrument wird eine Schneidfunktion demnach mechanisch bewirkt.

Der Erfindung liegt allgemein die Aufgabe zugrunde, ein bipolares Instrument zum Schneiden von Gewebe der eingangs genannten Art ausgehend von Dokument WO-A-99/23960 weiter zu entwickeln.

Erfindungsgemäß wird diese Aufgabe durch ein medizinisches bipolares Instrument gemäß Anspruch 1 gelöst.

Dabei ist es bevorzugt, wenn das stabförmige Element bzw. die stabförmigen Elemente als Stromzuführung ausgebildet ist/sind.

Hierdurch wird ein konstruktiv besonders einfacher Aufbau des erfindungsgemäßen Instruments erreicht, da für die Stromzuführung keine separaten Leitungen oder Drähte erforderlich sind. Das zumindest eine stabförmige Element bzw. die stabförmigen Elemente können am proximalen Ende mit einem einfachen Handgriff als Handhabe ausgestattet sein.

Es ist von Vorteil, daß die Schnittlinie, die durch die sich gegenüberstehenden Vorsprünge definiert wird, sehr exakt definiert ist. Die Vorsprünge können sich dabei in Längsrichtung der Arbeitselemente oder in einer Schräg- oder Querrichtung gegenüberstehen. Bspw. kann bei sich in Querrichtung der Arbeitselemente gegenüberstehenden Vorsprüngen durch axiales Verschieben des Instruments oder durch eine Bewegung quer zur Längsrichtung der Arbeitselemente geschnitten werden, und bei sich in Längsrichtung gegenüberstehenden Vorsprüngen kann durch eine Drehung des Instruments um seine Längsachse geschnitten werden.

Bei sich in Arbeitselementlängsrichtung gegenüberstehenden Vorsprüngen ist es weiterhin bevorzugt, wenn das erste Arbeitselement das zweite Arbeitselement in Arbeitselementlängsrichtung überragt.

Es kann ein besonders scharfer und wohldefinierter kurzer Schnitt gesetzt werden, da das freie Ende des zumindest einen Vorsprungs des ersten Arbeitselements und das freie Ende des zumindest einen Vorsprungs des zweiten Arbeitselements einen minimalen Abstand voneinander aufweisen, da der Stromfluß somit optimal auf die kurze Verbindungsstrecke zwischen den Vorsprüngen mit hoher Dichte konzentriert ist. "Minimaler" Abstand bedeutet, daß der Abstand der Arbeitselemente im Bereich der Vorsprünge kleiner ist als im übrigen Bereich der Arbeitselemente.

Ein solcher minimaler Abstand kann bspw. etwa 1 mm oder darunter betragen.

Eine besonders vorteilhafte Anpassung des erfindungsgemäßen Instruments an den jeweiligen Anwendungszweck wird in diesem Sinne dadurch erreicht, daß das erste Arbeitselement und das zweite Arbeitselement auswechselbar sind. Es können demnach verschiedene Arbeitselemente mit einer entsprechenden Anzahl und Verteilung von Vorsprüngen bereitgehalten werden.

Weitere Ausführungsbeispiele werden in den abhängigen Ansprüchen definiert.

Weitere Vorteile ergeben sich aus der nachfolgenden Beschreibung und der beigefügten Zeichnung.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

In der Zeichnung zeigen:
- Fig. 1: ein medizinisches bipolares Instrument zum Schneiden von Gewebe in einer Gesamtdarstellung in Seitenansicht in zwei Teilbildern, wobei ein distaler Abschnitt des Instruments im vergrößerten Maßstab dargestellt ist;
- Fig. 2: eine perspektivische Darstellung des distalen Abschnitts des Instruments in Fig. 1 in einem noch weiter vergrößerten Maßstab;
- Fig. 3: ein weiteres Ausführungsbeispiel für ein erstes und zweites Arbeitselement des Instruments in Fig. 1 in einer perspektivischen Darstellung in stark vergrößertem Maßstab;
- Fig. 4: ein noch weiteres Ausführungsbeispiel für ein erstes und zweites Arbeitselement für das Instrument in Fig. 1 in perspektivischer Darstellung in stark vergrößertem Maßstab;
- Fig. 5a) und b): eine schematische Draufsicht (Fig. 5a)) und Vorderansicht (Fig. 5b)) des ersten und zweiten Arbeitselements aus Fig. 2;
- Fig. 6a) und b): eine schematische Draufsicht (Fig. 6a)) und Vorderansicht (Fig. 6b)) des ersten und zweiten Arbeitselements aus Fig. 4;
- Fig. 7a) und b): eine schematische Draufsicht (Fig. 7a)) und Seitenansicht (Fig. 7b)) eines noch weiteren Ausführungsbeispiels eines ersten und zweiten Arbeitselements zur Verwendung in dem Instrument gemäß Fig. 1;
- Fig. 8: ein gegenüber Fig. 7 abgewandeltes Ausführungsbeispiel für ein erstes und zweites Arbeitselements in einer Draufsicht;
- Fig. 9: ein weiteres Ausführungsbeispiel eines bipolaren Instruments zum Schneiden von Gewebe im Bereich seines distalen Endes in Seitenansicht;
- Fig. 10: das Instrument in Fig. 9, wobei das eine Arbeitselement weggelassen wurde;
- Fig. 11: eine Draufsicht auf das distale Ende des Instruments in Fig. 9 und 10;
- Fig. 12: eine schematische Darstellung eines noch weiteren Ausführungsbeispiels eines bipolaren Instruments zum Schneiden von Gewebe in Draufsicht;
- Fig. 13: ein gegenüber Fig. 12 abgewandeltes Ausführungsbeispiel eines weiteren Instruments zum Schneiden von Gewebe, wobei das Instrument nur im Bereich seiner Arbeitselemente dargestellt ist; und
- Fig. 14: ein noch weiteres Ausführungsbeispiel für ein Instrument zum Schneiden von Gewebe, wobei das Instrument nur im Bereich seiner Arbeitselemente dargestellt ist.

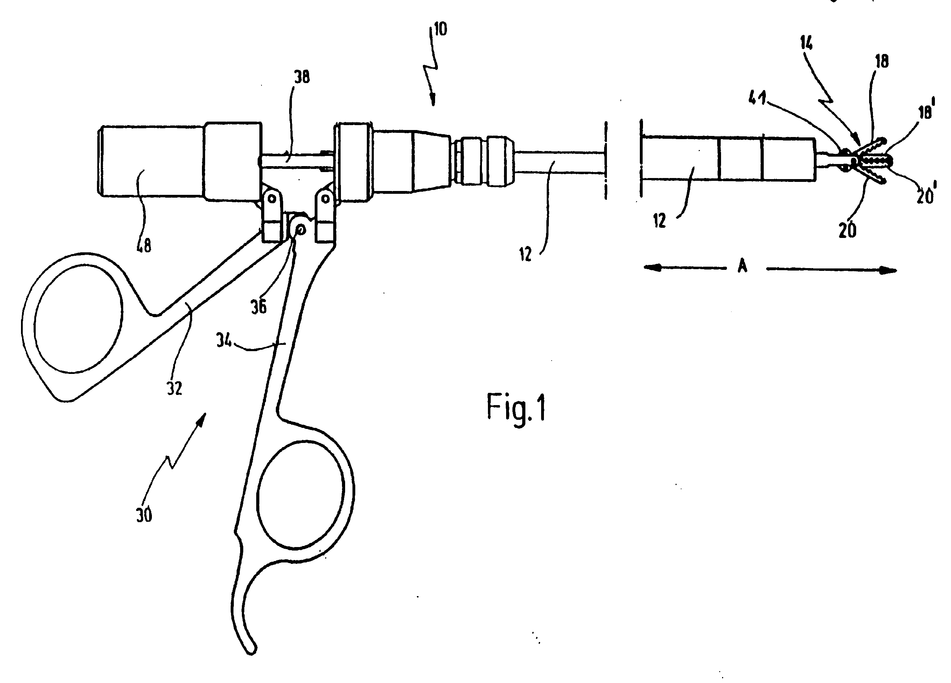

Die in den Fig. 1 bis 11 dargestellten Ausführungsbeispiele dienen lediglich der Erläuterung der in den Fig. 12 bis 14 dargestellten erfindungsgemäßen Ausführungsbeispiele. Die Fig. 12 bis 14 sind in der vorliegenden Anmeldung nicht enthalten.

In Fig. 1 ist ein mit dem allgemeinen Bezugszeichen 10 versehenes medizinisches bipolares Instrument zum Schneiden von Gewebe dargestellt. Mit dem Instrument 10 kann, wie nachfolgend näher beschrieben wird, Gewebe nicht nur geschnitten, sondern auch gefaßt und koaguliert werden. Das Instrument 10 wird im Rahmen der minimal-invasiven Chirurgie zum Präparieren von Gewebe verwendet.

Das Instrument 10 weist einen langerstreckten Schaft 12 auf, dessen in Fig. 1 mit A bezeichneter distaler Abschnitt in vergrößertem Maßstab dargestellt ist.

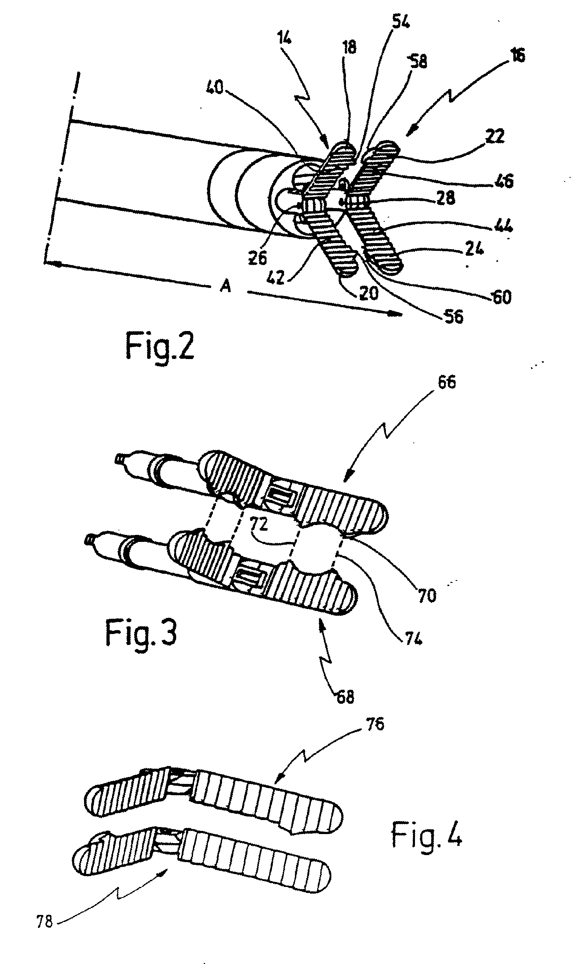

Der Abschnitt A ist in Fig. 2 perspektivisch und noch weiter vergrößert dargestellt.

Der Schaft 12 ist als Rohrschaft ausgebildet. Am distalen Ende des Schaftes 12 sind ein erstes Arbeitselement 14 und ein dazu benachbartes zweites Arbeitselement 16 angeordnet, wobei in der Seitenansicht in Fig. 1 nur das rechte erste Arbeitselement 14 zu sehen ist. Die Arbeitselemente 14 und 16 sind jeweils als Maul ausgebildet.

Das erste Arbeitselement 14 weist ein erstes Maulteil 18 und ein zweites Maulteil 20 auf.

Das zweite Arbeitselement 16 weist entsprechend ein erstes Maulteil 22 und ein zweites Maulteil 24 auf.

Das erste Maulteil 18 und das zweite Maulteil 20 sind relativ zueinander beweglich. In dem in Fig. 2 gezeigten Ausführungsbeispiel sind dabei sowohl das erste Maulteil 18 als auch das zweite Maulteil 20 beweglich, und zwar sind beide um eine Schwenkachse 26 verschwenkbar. Ebenso sind das erste Maulteil 22 und das zweite Maulteil 24 des zweiten Arbeitselements 16 relativ zueinander beweglich, und zwar sind beide Maulteile 22 und 24 um eine Schwenkachse 28 verschwenkbar.

In Fig. 1 ist das Arbeitselement 14 mit seinen Maulteilen 18 und 20 in Offenlage und mit 18' und 20' in seiner Schließlage dargestellt.

Wie aus Fig. 2 hervorgeht, sind das erste Arbeitselement 14 und das zweite Arbeitselement 16 benachbart, und zwar Seite an Seite voneinander beabstandet angeordnet. Speziell sind das erste Arbeitselement 14 und das zweite Arbeitselement 16 parallel zueinander ausgerichtet und lassen sich synchron öffnen und schließen.

Zum Öffnen und Schließen des ersten Arbeitselements 14 und des zweiten Arbeitselements 16 weist das Instrument 10 eine Handhabe 30 auf, die zwei relativ zueinander bewegliche Griffteile 32 und 34 aufweist. Das Griffteil 32 und das Griffteil 34 sind dazu über eine Schwenkachse 36 gelenkig miteinander verbunden.

Das Griffteil 32 ist über einen Kraftübertragungsmechanismus, der zwei Zug- und Schubstangen umfaßt, von denen in Fig. 1 nur die rechte Zug- und Schubstange 38 zu sehen ist, mit dem ersten Arbeitselement 14 bzw. dem zweiten Arbeitselement 16 kraftschlüssig verbunden. Die Zug- und Schubstange 38 sowie die parallel dazu verlaufende, in Fig. 1 nicht sichtbare Schub- und Zugstange sind jeweils über eine Kniehebelanordnung 41 mit dem entsprechenden Arbeitselement 14 bzw. 16 verbunden.

Durch Zusammendrücken des ersten Griffteils 32 und des zweiten Griffteils 34 werden das das erste Arbeitselement 14 bildende Maul und das das zweite Arbeitselement 16 bildende Maul synchron geschlossen, und durch Auseinanderbewegen der Griffteile 32 und 34 werden das erste Maul und das zweite Maul synchron geöffnet.

Gemäß einer ersten Funktion des ersten Arbeitselements 14 und des zweiten Arbeitselements 16 sind die Maulteile 18 und 20 bzw. 22 und 24 als Faßwerkzeuge ausgebildet, d.h. Innnenseiten 40, 42, 44 und 46 der entsprechenden Maulteile 18, 20, 22 und 24 sind flächig ausgebildet und zusätzlich mit Zahnungen versehen, um die Griffigkeit der Innenseiten zum Fassen und sicheren Festhalten von Gewebe zu verbessern.

Gemäß einer weiteren Funktion des ersten Arbeitselements 14 und des zweiten Arbeitselements 16 ist das erste Arbeitselement 14 als mit Hochfrequenzstrom beaufschlagbare Elektrode und das zweite Arbeitselement 16 als Gegenelektrode ausgebildet, um zwischen den Maulteilen 18 und 20 bzw. 22 und 24 gefaßtes Gewebe zu koagulieren. Die Maulteile 18 und 20 bzw. 22 und 24 sind dazu zumindest an ihren Innenseiten 40 bis 46 oder insgesamt metallisch und damit stromführend ausgebildet.

Das erste Arbeitselement 14 ist über eine Stromzuführung, die durch die zugehörige Zug- und Schubstange gebildet ist, mit einem ersten nicht näher dargestellten Kontaktpol eines Steckeranschlusses 48 am proximalen Ende des Instruments 10 in Fig. 1 verbunden, während das zweite Arbeitselement 16 über die andere Zug- und Schubstange als Stromzuführung mit dem anderen Kontaktpol des Steckeranschlusses 48 verbunden ist. An den Steckeranschluß 48 ist ein Hochfrequenzstromkabel (nicht dargestellt) anschließbar, das mit einer externen nicht dargestellten Hochfrequenzspannungsquelle verbindbar oder verbunden ist.

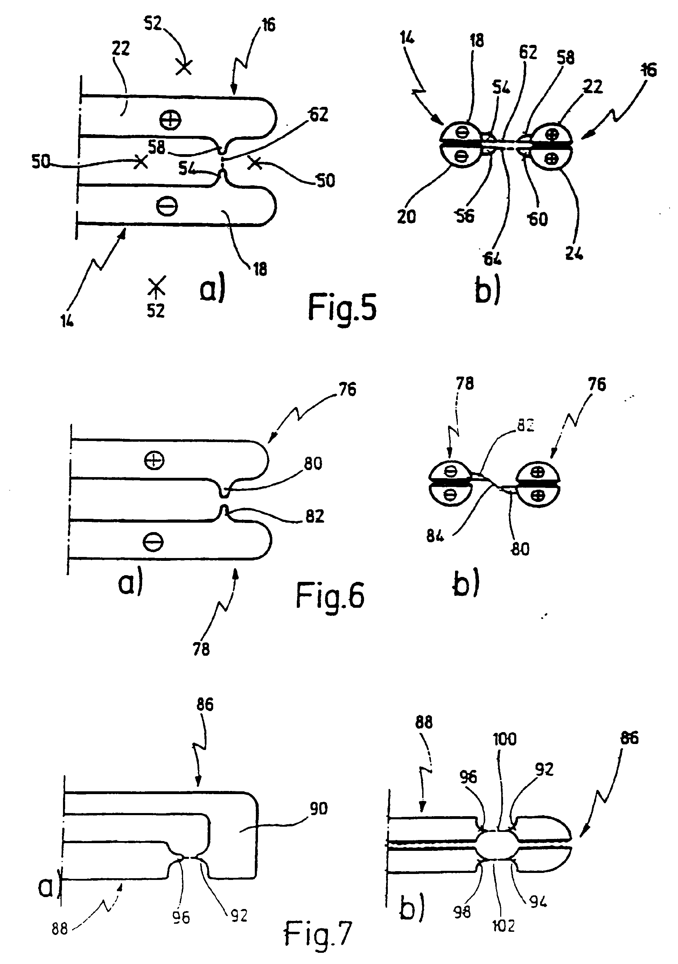

In Fig. 5, die das erste Arbeitselement 14 und das zweite Arbeitselement 16 in Alleinstellung schematisch darstellt, ist mit einem + angedeutet, daß das zweite Arbeitselement 16 die eine Elektrode und mit einem - angedeutet, daß das erste Arbeitselement 14 die Gegenelektrode bildet, d.h. das Arbeitselement 16 und das Arbeitselement 14 befinden sich auf unterschiedlichen Potentialen.

Da das erste Arbeitselement 14 und das zweite Arbeitselement 16 voneinander beabstandet sind, sind zwischen dem ersten Arbeitselement 14 und dem zweiten Arbeitselement 16 keine Isolierungsmaßnahmen notwendig, wodurch sich die Anordnung mit zwei Seite an Seite voneinander beabstandet angeordneten Arbeitselementen 14 und 16 für schmal bauende bipolare Instrumente besonders bewährt hat.

Beim Beaufschlagen des ersten Arbeitselements 14 und des zweiten Arbeitselements 16 mit Strom kann somit zwischen den Innenseiten 40 und 42 bzw. 44 und 46 gefaßtes Gewebe unter der Wirkung des Hochfrequenzstroms koaguliert werden, wobei die Koagulationswirkung auf einen Zwischenraum 50 zwischen dem ersten Arbeitselement 14 und dem zweiten Arbeitselement 16 beschränkt ist, so daß außerhalb dieses Bereichs befindliches Gewebe durch den Hochfrequenzstrom nicht beeinträchtigt wird. In Bereichen, die außerhalb des ersten Arbeitselements 14 und des zweiten Arbeitselements liegen, wie mit 52 angedeutet, fließt kein Strom, und entsprechend tritt dort auch keine Koagulation von Gewebe auf.

Das Instrument 10 weist nun eine dritte Funktion auf, nämlich können mit dem Instrument 10 mit dem ersten Arbeitselement 14 und dem zweiten Arbeitselement 16 gefaßtes Gewebe und auch Gefäße, die in dem Gewebe vorhanden sind, elektrisch unter der Wirkung des Hochfrequenzstroms geschnitten bzw. durchtrennt werden.

Dazu weist zumindest eines der Arbeitselemente 14 oder 16, im Ausführungsbeispiel gemäß Fig. 2 sowohl das erste Arbeitselement 14 als auch das zweite Arbeitselement 16, jeweils zumindest einen, im Ausführungsbeispiel gemäß Fig. 2 jeweils zwei Vorsprünge auf. Dabei weisen das Maulteil 18 des Arbeitselements 14 und das Maulteil 22 des Arbeitselements 16 jeweils einen gegenüberliegenden Vorsprung und das Maulteil 20 des Arbeitselements 14 und das Maulteil 24 des Arbeitselements 16 einen gegenüberliegenden Vorsprung auf, wobei der Vorsprung des Maulteils 18 und der Vorsprung des Maulteils 20 desselben Arbeitselements 14 in Arbeitselementlängsrichtig auf gleicher Höhe und ebenso der Vorsprung der Maulteile 22 und 24 des zweiten Arbeitselements 16 in Arbeitselementlängsrichtung auf gleicher Höhe liegen.

Ein erster Vorsprung 54 ist an dem ersten Maulteil 18, und ein zweiter Vorsprung 56 ist an dem zweiten Maulteil 20 des ersten Arbeitselements 14, und ein erster Vorsprung 58 ist an dem ersten Maulteil 22 und ein zweiter Vorsprung 60 an dem zweiten Maulteil 24 des zweiten Arbeitselements 16 angeordnet.

Die Vorsprünge 54 und 56 des ersten Arbeitselements 14 sind zu dem zweiten Arbeitselement 16 hin gerichtet, und in umgekehrter Weise sind die Vorsprünge 58 und 60 zu dem ersten Arbeitselement 14 hin gerichtet.

Jedes freie Ende der Vorsprünge 54 bis 60 ist nun derart ausgebildet, daß an diesen freien Enden eine Konzentration der Stromdichte des Hochfrequenzstroms auftritt. Dies wird durch die Form der Vorsprünge 54 bis 60, genauer gesagt der freien Enden dieser Vorsprünge 54 bis 60, erreicht, indem die freien Enden der Vorsprünge 54 bis 60 eine Spitze aufweisen, so daß die elektrische Feldstärke an den Spitzen im Vergleich zu den übrigen Bereichen des ersten Arbeitselements 14 und des zweiten Arbeitselements 16 ein Maximum erreicht.

Die Vorsprünge 54 bis 60 sind dabei so positioniert, daß die Vorsprünge 54 und 58 und die Vorsprünge 56 und 60 sich unmittelbar mit einem minimalen Abstand gegenüberstehen.

In Fig. 5 sind mit gestrichelten Linien 62 und 64 die Stromlinien mit maximaler Konzentration der Stromdichte angedeutet. Entlang dieser Stromlinien können mit dem ersten Arbeitselement 14 und dem zweiten Arbeitselement 16 Gewebe und darin enthaltene Gefäße unter Wirkung des Hochfrequenzstroms aufgrund der maximalen Konzentration der Stromdichte geschnitten werden. Zum Schneiden eines Gefäßes wird das Instrument 10 so gehalten, daß die Stromlinien quer zur Längsachse des Gefäßes verlaufen.

Bei dem in Fig. 5 gezeigten Ausführungsbeispiel sind die Vorsprünge 54 bis 60 quer zur Maullängsrichtung des ersten Arbeitselements 14 und des zweiten Arbeitselements 16 gerichtet, wobei das Instrument 10 zum Schneiden eines Gefäßes so gehalten wird, daß sich das Gefäß zwischen den Arbeitselementen 14 und 16 befindet und sich parallel zu diesen erstreckt.

Des weiteren sind alle vier Vorsprünge 54 bis 60 in Maullängsrichtung auf gleicher Höhe angeordnet. Die Positionierung der Vorsprünge 54 und 56 bzw. 58 und 60 ermöglicht ein Schneiden des Gewebes von beiden Seiten her, und zwar entlang der definierten Schnittlinien 62 und 64.

Es versteht sich, daß die Vorsprünge 54 bis 60 metallisch ausgebildet sind und mit dem jeweiligen Maulteil 18 bis 24 elektrisch leitend verbunden sind. Die Vorsprünge 54 bis 60 können bei der Herstellung der Maulteile 18 bis 24 in einem materialabtragenden Formgebungsverfahren ausgebildet werden, wenn die Maulteile 18 bis 24 aus einem Vollmaterial herausgearbeitet werden.

Mit dem Instrument 10 und den Arbeitselementen 14 und 16 gemäß Fig. 5a und 5b wird Gewebe wie folgt präpariert.

Bei geöffneten Maulteilen 18 und 20 bzw. 22 und 24 wird zunächst das zu präparierende Gewebe zwischen die Maulteile 18 und 20 bzw. 22 und 24 gebracht, wonach die Maulteile 18 und 20 bzw. 22 und 24 geschlossen werden.

Unter der Wirkung des bipolaren Hochfrequenzstroms, der bei angeschlossener und eingeschalteter Hochfrequenzspannungsquelle zwischen dem ersten Arbeitselement 14 und dem zweiten Arbeitselement 16 fließt, wird das mit den Maulteilen 18 und 20 bzw. 22 und 24 gefaßte Gewebe koaguliert. Soll ein Gefäß in dem Gewebe koaguliert werden, wird das Gewebe so gefaßt, daß sich das Gefäß quer zu den Arbeitselementen 14 und 16 erstreckt.

Um das Gewebe anschließend zu schneiden oder ein darin befindliches Gefäß zu durchtrennen, werden die Maulteile 18 und 20 bzw. 22 und 24 wieder geöffnet, und das Instrument 10 wird insgesamt etwas zurückgezogen, um das gefaßte Gewebe loszulassen und anschließend werden die Maulteile 18 und 20 bzw. 22 und 24 geschlossen.

Hiernach wird das Instrument 10 um etwa 90° um seine Längsachse gedreht, wonach dann mit geschlossenen Maulteilen 18 und 20 bzw. 22 und 24 durch Vorschieben des Instruments 10 mittels der die sich zwischen den Vorsprüngen 54 und 58 bzw. 56 und 60 ausbildenden Schnittlinien 62 und 64 geschnitten werden kann.

Aus der vorstehenden Beschreibung ergibt sich, daß das Instrument 10 aufgrund der Ausgestaltung des ersten Arbeitselements 14 und des zweiten Arbeitselements 16 die drei Funktionen Fassen, Koagulieren und Schneiden von Gewebe in sich vereint.

Die Vorsprünge 54 und 58 bzw. 56 und 60 sind jeweils mit ihren freien Enden minimal, etwa 1 mm, voneinander beabstandet.

Dadurch, daß sowohl an dem ersten Maulteil 18 als auch an dem zweiten Maulteil 20 jeweils ein Vorsprung 54 bzw. 56 vorgesehen ist, ebenso wie an den Maulteilen 22 und 24 des zweiten Arbeitselements 16, wird zwischen dem ersten Arbeitselement 14 und dem zweiten Arbeitselement 16 eine "elektrische Schneide" ausgebildet, die eine Höhe aufweist, die etwa dem Abstand zwischen den Vorsprüngen 54 und 56 einerseits und den Vorsprüngen 58 und 60 andererseits entspricht.

In Fig. 3 ist ein gegenüber Fig. 2 abgewandeltes Ausführungsbeispiel für ein erstes Arbeitselement 66 und ein zweites Arbeitselement 68 dargestellt, die jeweils wie die Arbeitselemente 14 und 16 als Maul ausgebildet sind, wobei sowohl das erste Arbeitselement 66 als auch das zweite Arbeitselement 68 jeweils vier Vorsprünge 70, d.h. insgesamt acht Vorsprünge 70, aufweisen. Bei dieser Ausgestaltung ergeben sich in Arbeitselementlängsrichtung gesehen zwei voneinander beabstandete Schnittlinien 72 und 74.

Ein weiterer Unterschied zwischen dem ersten Arbeitselement 66 und dem zweiten Arbeitselement 68 gemäß Fig. 3 und dem ersten Arbeitselement 14 und dem zweiten Arbeitselement 16 gemäß Fig. 2 besteht darin, daß das erste Arbeitselement 66 und das zweite Arbeitselement 68 nur jeweils ein bewegliches Maulteil und jeweils ein unbewegliches Maulteil aufweisen, wodurch die jeweiligen Maulteile jedoch wiederum relativ zueinander beweglich sind, wie im Ausführungsbeispiel gemäß Figuren 2 und 1.

In Figuren 4 und 6 ist ein noch weiteres Ausführungsbeispiel für ein erstes Arbeitselement 76 und ein zweites Arbeitselement 78 dargestellt.

Bei diesem Ausführungsbeispiel weisen das erste Arbeitselement 76 und das zweite Arbeitselement 78 jeweils nur einen Vorsprung 80 und 82 auf, wobei der Vorsprung 80 und der Vorsprung 82 jeweils an einem Maulteil des ersten Arbeitselements 76 und des zweiten Arbeitselements 78 angeordnet sind, die sich diametral gegenüberliegen. Auf diese Weise wird eine Schnittlinie 84 erzeugt, die bezüglich der Hauptebene des ersten Arbeitselements 76 und des zweiten Arbeitselements 78 schräg verläuft, d.h. bezüglich der Horizontalen geneigt.

Ein weiteres Ausführungsbeispiel für ein erstes Arbeitselement 86 und ein zweites Arbeitselement 88 ist in Fig. 7 dargestellt, das sich von den vorherigen Ausführungsbeispielen dadurch unterscheidet, daß das erste Arbeitselement 86 das zweite Arbeitselement 88 in Arbeitselementlängsrichtung überragt. Das erste Arbeitselement 86 und das zweite Arbeitselement 88 sind weiterhin Seite an Seite voneinander beabstandet angeordnet, jedoch ist das erste Maul 86 L-förmig ausgebildet, wobei ein quer zur Arbeitselementlängsrichtung stehender Abschnitt 90 vor dem äußersten distalen Ende des Arbeitselements 88 angeordnet ist.

Das erste Arbeitselement 86 und das zweite Arbeitselement 88 weisen jeweils zwei Vorsprünge 92 und 94 bzw. 96 und 98 auf, die im Unterschied zu den vorherigen Ausführungsbeispielen in Arbeitselementlängsrichtung gerichtet sind, so daß Schnittlinien 100 und 102 ebenfalls in Arbeitselementlängsrichtung gerichtet sind. Mit dieser Anordnung der Vorsprünge -92 bis 98 läßt sich mit dem ersten Arbeitselement 86 und dem zweiten Arbeitselement 88 ein Gefäß, das sich quer zur Arbeitselementlängsrichtung erstreckt, durch Drehen des Instruments um seine Längsachse oder durch Bewegen des Arbeitselements in Richtung quer zur Längsrichtung durchtrennen.

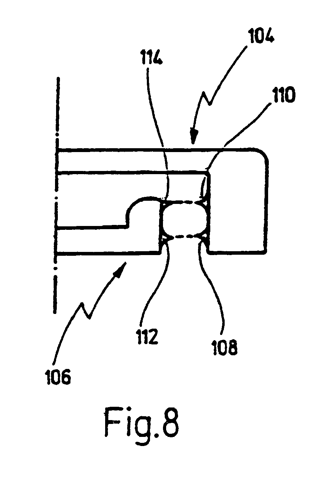

Fig. 8 zeigt ein gegenüber der Fig. 7 leicht abgewandeltes Ausführungsbeispiel, bei dem jedes Maulteil des ersten Arbeitselements 104 und jedes Maulteil des zweiten Arbeitselement 106 zwei nebeneinander angeordnete Vorsprünge 108 und 110 bzw. 112 und 114 aufweist.

Alle die vorgenannten Ausführungsbeispiele können bei dem Instrument 10 in Fig. 1 Verwendung finden. Dazu ist es bei dem Instrument 10 vorgesehen, das erste Arbeitselement 14 und das zweite Arbeitselement 16 abnehmbar auszugestalten, um so anstelle des ersten Arbeitselements 14 und des zweiten Arbeitselements 16 die in Figuren 3 bis 8 dargestellten Mäuler 66, 68; 76, 78; 86, 88 oder 104 und 106 zu montieren.

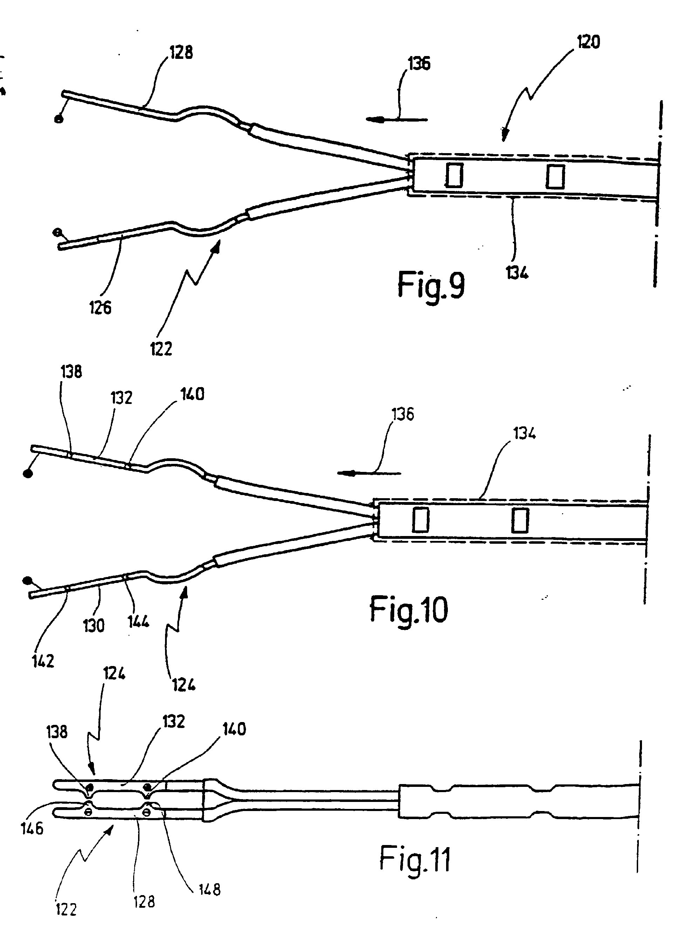

In Fig. 9 bis 11 ist ein weiteres Ausführungsbeispiel für ein bipolares Instrument zum Schneiden von Gewebe dargestellt, wobei das Instrument 120 wie das Instrument 10 ebenfalls die Funktionen Fassen, Koagulieren und Schneiden in sich vereint.

Das Instrument 120 weist ein erstes Arbeitselement 122 sowie ein zweites Arbeitselement 124 auf, die jeweils als Maul ausgebildet sind. In Fig. 9 ist die Außenseite des ersten Arbeitselements 122 (linkes Arbeitselement) zu sehen, während in Fig. 10 die Innenseite des zweiten Arbeitselements 124 (rechtes Arbeitselement) zu sehen ist.

Das erste Arbeitselement 122 weist ein erstes Maulteil 126 und ein zweites Arbeitselement 128 auf. Ebenso weist das zweite Arbeitselement 124 ein erstes Maulteil 130 und ein zweites Maulteil 132 auf.

Das Maulteil 126 und das Maulteil 128 des ersten Arbeitselements 122 sind relativ zueinander beweglich, d.h. können aus der in Fig. 9 dargestellten Offenlage in eine Schließlage aufeinander zu bewegt werden. Entsprechendes gilt für die Maulteile 130 und 132 des zweiten Arbeitselements 124.

Der Unterschied zwischen dem ersten Arbeitselement 122 bzw. zweiten Arbeitselement 124 und dem ersten Arbeitselement 14 bzw. dem zweiten Arbeitselement 16 des Instruments 10 besteht darin, daß die Maulteile 18 und 20 bzw. 22 und 24 des Instruments 10 über ein Gelenk miteinander verbunden sind, während die Maulteile 126 und 128 bzw. 130 und 132 als sich federnd aufspreizende Elemente ausgebildet sind, die durch eine Relativbewegung zu einem in Fig. 9 und 10 mit unterbrochenen Linien angedeuteten Schieberohr 134 zusammengedrückt werden können. Bei einer Bewegung des Schieberohrs 134 relativ zu den Maulteilen 126 und 128 bzw. 130 und 132 in Richtung eines Pfeils 136 werden die Maulteile 126 bis 132 geschlossen, und öffnen sich selbsttätig wieder durch eine umgekehrte Bewegung des Schieberohrs 134 relativ zu den Maulteilen 126 bis 132. Dieser Öffnungs- und Schließmechanismus ist bereits bspw. aus dem deutschen Gebrauchsmuster 298 23 913 bekannt, auf das hinsichtlich weiterer Einzelheiten hinsichtlich des Bewegungsmechanismus verwiesen wird.

Ähnlich zur Ausgestaltung in Fig. 3 weisen die Maulteile 126 bis 132 auf ihren einander gegenüberliegenden Seiten jeweils zwei Vorsprünge 138 und 140 (Maulteil 132), 142 und 144 (Maulteil 130), 146 und 148 (Maulteil 128) sowie zwei entsprechende, in den Figuren nicht sichtbare Vorsprünge am Maulteil 126 auf. Die Arbeitselemente 122 und 124 bilden eine Elektrode und eine Gegenelektrode, wie mit ⊕ und ⊖ angedeutet ist.

Hinsichtlich der Schneidfunktion des Instruments 120 entspricht die Ausgestaltung der Arbeitselemente 122 und 124 den Arbeitselementen 66 und 68 in Fig. 3, so daß auf die dortigen Ausführungen verwiesen werden kann.

In Fig. 12 bis 14 sind weitere Ausführungsbeispiele für bipolare Instrumente zum Schneiden von Gewebe dargestellt. Diese Instrumente unterscheiden sich von den vorherigen Instrumenten dadurch, daß das erste und das zweite Arbeitselement nicht als Maul ausgebildet sind, sondern daß die Arbeitselemente der nachfolgenden Instrumente unbeweglich sind, so daß diese Instrumente eine reine Schneidfunktion, ggf. mit einer Koagulationsfunktion, besitzen, jedoch keine Faßfunktion.

In Fig. 12 ist zunächst ein bipolares Instrument 160 zum Schneiden von Gewebe schematisch dargestellt.

Das Instrument 160 weist ein erstes Arbeitselement 162 und ein zweites Arbeitselement 164 auf, wobei das erste Arbeitselement 162 und das zweite Arbeitselement 164 wiederum zueinander benachbart sind.

Das Arbeitselement 162 und das Arbeitselement 164 sind nicht wie zuvor als Maul ausgebildet, sondern als unbewegliche Elemente, die am distalen Ende jeweils eines stabförmigen Elements 166 bzw. 168 in einstückiger Bauweise mit diesen angeordnet sind.

Die stabförmigen Elemente 166 bzw. 168 dienen gleichzeitig als Stromzuführungen von einem proximalen, nicht dargestellten Hochfrequenzstromanschluß, der vorzugsweise am proximalen Ende einer Handhabe 170 angeordnet ist.

Das erste Arbeitselement 162 bildet entsprechend eine Elektrode, und das zweite Arbeitselement 164 eine Gegenelektrode des Instruments 160.

Das erste Arbeitselement 162 weist auf seiner dem zweiten Arbeitselement 164 zugewandten Seite zwei Vorsprünge 172 und 174 auf, während das zweite Arbeitselement 164 den Vorsprüngen 172 und 174 gegenüberstehend ebenfalls zwei Vorsprünge 176 und 178 aufweist.

Die stabförmigen Elemente 166 und 168 nebst den Arbeitselementen 162 und 164 können dabei vollständig umfänglich mit einer elektrischen Isolierung versehen sein, so daß nur die Vorsprünge 172 bis 178 metallisch blank sind. Es kann jedoch auch vorgesehen sein, die Arbeitselemente 162 und 164 in den mit 180 und 182 bezeichneten Bereichen ebenfalls metallisch blank auszugestalten, so daß nur die stabförmigen Elemente 166 und 168 eine elektrisch isolierende Umhüllung aufweisen.

Mit dem Instrument 160 wird ein "elektrisches Messer" bereitgestellt, mit dem Gewebe geschnitten bzw. Gefäße durchtrennt werden können.

Für den Fall, daß die Bereiche 180 und 182 der Arbeitselemente 162 und 164 zumindest teilweise metallisch blank sind, kann das Instrument 160 auch zum Koagulieren mit den flächigen Bereichen der Arbeitselemente 162 und 164 verwendet werden.

In Fig. 13 ist ein gegenüber Fig. 12 geringfügig abgewandeltes Ausführungsbeispiel eines Instruments 200 dargestellt, dessen Arbeitselemente 202 und 204 jeweils einen Vorsprung 206 bzw. 208 aufweisen, die als längliche Kanten ausgebildet sind.

Die länglichen Kanten der Vorsprünge 206 und 208 verlaufen dabei schräg zur Arbeitselementlängsrichtung, wie aus Fig. 13 hervorgeht. Die Orientierung der länglichen Kante des Vorsprunges 206 ist dabei gegensinnig zur Orientierung der länglichen Kante des Vorsprungs 208, wie ebenfalls aus Fig. 13 ersichtlich ist.

Auch das in Fig. 13 dargestellte Instrument 200 dient als Messer mit elektrischer Schneidfunktion.

Schließlich ist in Fig. 14 ein bipolares Instrument 220 zum Schneiden von Gewebe dargestellt, mit einem ersten Arbeitselement 222 und einem zweiten Arbeitselement 224, wobei das erste Arbeitselement 222 das zweite Arbeitselement 224 axial überragt, ähnlich wie die in Fig. 7 dargestellten Arbeitselemente 86 und 88.

Das erste Arbeitselement 222 weist einen Vorsprung 226 und das zweite Arbeitselement 224 einen Vorsprung 228 auf, wobei sich die Vorsprünge 226 und 228 in Arbeitselementlängsrichtung gegenüberstehen.

Mit dieser Ausgestaltung des Instruments 220 kann ein Schnitt in Gewebe oder in ein Gefäß auch dadurch eingebracht werden, daß das Instrument 220 um seine Längsachse gedreht, oder quer zur Längsrichtung des Instruments 220 bewegt wird.

Bei den Instrumenten 160 gemäß Fig. 12 bzw. 200 gemäß Fig. 13 kann ein Schnitt durch Vor- oder Zurückschieben des Instruments 160 bzw. 200 in deren Längsrichtung erfolgen, bspw. um ein Gefäß zu durchtrennen, das sich quer zur Längsrichtung der Arbeitselemente 162 und 164 bzw. 202 und 204 erstreckt, oder durch Bewegen der Instrumente in einer Richtung quer zu deren Längsrichtung, wenn sich das Gefäß parallel zu den Arbeitselementen erstreckt.

Im Rahmen der Erfindung ist es, wie die vorhergehenden Ausführungsbeispiele zeigen, möglich, durch entsprechende Wahl der Anzahl und Positionierung von Vorsprüngen der Anwendung entsprechende Schnittlinien zum elektrischen Schneiden von Gewebe und Gefäßen zu erzeugen.

Die in den gezeigten Ausführungsbeispielen dargestellte Form der Vorsprünge, die als warzenartig bezeichnet werden kann, und die Anzahl der Vorsprünge ist nur beispielhaft zu verstehen, und es können beliebige andere Formen und Anzahlen von Vorsprüngen gewählt werden, wenn diese geeignet sind, an ihrem freien Ende eine Konzentration der Stromdichte des Hochfrequenzstroms zu bewirken, um elektrisch schneiden zu können.

Die freien Enden der Vorsprünge können anstelle von scharfen Spitzen auch abgerundet sein, oder es können Vorsprünge mit klingenartigen länglichen Kanten vorgesehen sein.

## Patentansprüche

1. Medizinisches bipolares Instrument zum Schneiden von Gewebe, mit einem ersten Arbeitselement und zumindest einem zweiten Arbeitselement, die einander benachbart an einem distalen Ende des Instruments angeordnet sind, wobei das erste Arbeitselement und das zweite Arbeitselement als mit Hochfrequenzstrom beaufschlagbare Elektrode und Gegenelektrode ausgebildet sind, wobei das zweite Arbeitselement die eine Elektrode und das erste Arbeitselement die Gegenelektrode bildet, wobei das erste Arbeitselement und das zweite Arbeitselement unbeweglich am distalen Ende eines oder zweier stabförmiger Elemente angeordnet sind, wobei sowohl das erste Arbeitselement als auch das zweite Arbeitselement jeweils zumindest einen Vorsprung aufweisen, wobei der zumindest eine Vorsprung des ersten Arbeitselements zu dem zumindest einen Vorsprung des zweiten Arbeitselements hin gerichtet ist und diesem gegenübersteht, wobei das freie Ende des ersten Vorsprungs und das freie Ende des zweiten Vorsprungs derart ausgebildet sind, daß an diesen eine Konzentration der Stromdichte auftritt, und wobei das freie Ende des zumindest einen Vorsprungs des ersten Arbeitselements und das freie Ende des zumindest einen vorsprungs des zweiten Arbeitselements einen Abstand voneinander aufweisen, der kleiner ist als der Abstand des ersten Arbeitselements vom zweiten Arbeitselement im übrigen Bereich des ersten und zweiten Arbeitselements.

2. Instrument nach Anspruch 1, wobei der zumindest eine vorsprung des ersten und zweiten Arbeitselements schräg zur Arbeitselementlängsrichtung gerichtet ist.

3. Instrument nach Anspruch 1 oder 2, wobei das erste Arbeitselement das zweite Arbeitselement in Arbeitselementlängsrichtung überragt, und daß der zumindest eine Vorsprung des ersten und zweiten Arbeitselements in Arbeitselementlängsrichtung gerichtet ist.

4. Instrument nach einem der Ansprüche 1 bis 3, wobei das freie Ende des zumindest einen Vorsprungs des ersten und zweiten Arbeitselements als Spitze ausgebildet ist.

5. Instrument nach einem der Ansprüche 1 bis 4, wobei das freie Ende des zumindest einen Vorsprungs des ersten und zweiten Arbeitselements abgerundet ist.

6. Instrument nach einem der Ansprüche 1 bis 5, wobei das freie Ende des zumindest einen Vorsprungs des ersten und zweiten Arbeitselements als längliche Kante ausgebildet ist.

7. Instrument nach Anspruch 6, wobei der zumindest eine Vorsprung keilartig schräg zur Arbeitselementlängsrichtung verläuft.

8. Instrument nach einem der Ansprüche 1 bis 7, wobei das stabförmige Element bzw. die stabförmigen Elemente als Stromzuführungen ausgebildet ist/sind.

9. Instrument nach einem der Ansprüche 1 bis 8, wobei die zumindest beiden Vorsprünge in Arbeitselementlängsrichtung auf der gleichen Höhe angeordnet sind.

10. Instrument nach einem der Ansprüche 1 bis 9, wobei das erste Arbeitselement und das zweite Arbeitselement auswechselbar sind.

## Claims

1. A medical bipolar instrument for cutting tissue, comprising a first working element and at least one second working element, which are arranged adjacent to one another at a distal end of the instrument, wherein the first working element and the second working element are configured as an electrode and a counter electrode supplyable with high frequency current, the second working element forming the one electrode and the first working element forming the counter electrode, the first working element and the second working element being immovably arranged at the distal end of one or two rod-shaped elements, the first working element as well as the second working element each have at least one projection, the at least one projection of the first working element being directed towards and opposing the at least one projection of the second working element, the free end of the first projection and the free end of the second projection being configured such that a concentration of the current density occurs at same, the free end of the at least one projection of the first working element and the free end of the at least one projection of the second working element having a distance from one another which is smaller than the distance of the first working element from the second working element in the remaining area of the first and second working elements.

2. The instrument of claim 1, wherein the at least one projection of the first and second working elements is directed to be inclined with respect to the axial direction of the working elements.

3. The instrument of claim 1 or 2, wherein the first working element extends beyond the second working element in axial direction of the working elements, and wherein the at least one projection of the first and second working elements is directed in axial direction of the working elements.

4. The instrument of any one of claims 1 through 3, wherein the free end of the at least one projection of the first and second working elements is configured as a point.

5. The instrument of any one of claims 1 through 4, wherein the free end of the at least one projection of the first and second working elements is rounded.

6. The instrument of any one of claims 1 through 5, wherein the free end of the at least one projection of the first and second working elements is configured as an elongated edge.

7. The instrument of claim 6, wherein the at least one projection runs in wedge-like manner at an inclination with respect to the axial direction of the working elements.

8. The instrument of any one of claims 1 through 7, wherein the rod-shaped element or the rod-shaped elements is/are configured as electric conductor(s).

9. The instrument of any one of claims 1 through 8, wherein the at least two projections are arranged at the same position in axial direction of the working elements.

10. The instrument of any one of claims 1 through 9, wherein the first working element and the second working element are exchangeable.

## Revendications

1. Instrument médical bipolaire pour découper des tissus, avec un premier élément de travail et au moins un deuxième élément de travail qui sont placés l'un près de l'autre sur une extrémité distale de l'instrument, le premier élément de travail et le deuxième élément de travail étant conformés en électrode et contre-électrode pouvant être exposées à un courant à haute fréquence, le deuxième élément de travail formant la première électrode et le premier élément de travail formant la contre-électrode, le premier élément de travail et le deuxième élément de travail étant disposés de manière fixe sur l'extrémité distale d'un ou de deux éléments en forme de tige, aussi bien le premier élément de travail que le deuxième élément de travail présentant chacun au moins une saillie, ladite au moins une saillie du premier élément de travail étant dirigée vers ladite au moins une saillie du deuxième élément de travail et lui faisant face, l'extrémité libre de la première saillie et l'extrémité libre de la deuxième saillie étant conformées de façon qu'il apparaisse sur celles-ci une concentration de la densité de courant, et l'extrémité libre de ladite au moins une saillie du premier élément de travail et l'extrémité libre de ladite au moins une saillie du deuxième élément de travail présentant entre elles une distance qui est inférieure à la distance entre le premier élément de travail et le deuxième élément de travail dans la partie restante du premier et du deuxième élément de travail.

2. Instrument selon la revendication 1, dans lequel ladite au moins une saillie du premier et du deuxième élément de travail est dirigée à l'oblique par rapport à la direction longitudinale de l'élément de travail.

3. Instrument selon la revendication 1 ou 2, dans lequel le premier élément de travail dépasse du deuxième élément de travail dans la direction longitudinale de l'élément de travail, et ladite au moins une saillie du premier et du deuxième élément de travail est dirigée dans la direction longitudinale de l'élément de travail.

4. Instrument selon l'une des revendications 1 à 3, dans lequel l'extrémité libre de ladite au moins une saillie du premier et du deuxième élément de travail est conformée en pointe.

5. Instrument selon l'une des revendications 1 à 4, dans lequel l'extrémité libre de ladite au moins une saillie du premier et du deuxième élément de travail est arrondie.

6. Instrument selon l'une des revendications 1 à 5, dans lequel l'extrémité libre de ladite au moins une saillie du premier et du deuxième élément de travail est conformée en arête oblongue.

7. Instrument selon la revendication 6, dans lequel ladite au moins une saillie s'étend en forme de coin de manière oblique par rapport à la direction longitudinale de l'élément de travail.

8. Instrument selon l'une des revendications 1 à 7, dans lequel l'élément en forme de tige ou les éléments en forme de tige est/sont conformé(s) en amenées de courant.

9. Instrument selon l'une des revendications 1 à 8, dans lequel lesdites au moins deux saillies sont disposées à la même hauteur dans la direction longitudinale de l'élément de travail.

10. Instrument selon l'une des revendications 1 à 9, dans lequel le premier élément de travail et le deuxième élément de travail sont échangeables.
